# EUROPEAN PATENT APPLICATION

(11) **EP 1 397 997 A1**
(43) Date of publication of application: **17.03.2004**
(21) Application number: 02019770.3
(22) Date of filing: 04.09.2002
(51) Int. Cl.: A61B 10/00

(54) **Detection device**

(71) Applicant: Friedrich-Alexander-Universität Erlangen-Nürnberg, 91054 Erlangen (DE)
(72) Inventor: Gröschl, Michael, 91054 Erlangen (DE); Rauh, Michael, 96158 Frensdorf (DE)
(74) Representative: VOSSIUS & PARTNER

(57) **Abstract**

The present invention relates to a device and a method for non-invasive in-situ concentration of substances contained in saliva for quantitative and qualitative analytics. According to the present invention, there is provided a device for non-invasive and in-situ concentration and detection of at least one substance contained in oral fluid, especially in saliva. In a preferred embodiment, the device comprises a base element having a cavity, said base element being in permeable connection with said cavity; at least one concentration element to be provided in said cavity, wherein said at least one concentration element has at least one binding element that binds said substance(s); said base element further comprising an opening to said cavity for locating and withdrawing said concentration element(s). The method and the device according to the present invention is/are advantageous in that it/they simplify the procedure of analysing the saliva and detecting and analysing substances contained therein. The device and the method, i.a., allow to be easily used with children and babies.

## Description

The present invention relates to a device and a method for non-invasive in-situ concentration of substances contained in saliva for quantitative and qualitative analytics.

Nowadays, non-invasive techniques are widely used in the field of diagnostics and analytics. These techniques overcome the most important disadvantages of invasive techniques such as, e.g., harming the patients body or fear of the patient. It is, for example, often not possible or not advisable to take blood during physical stress situations. Further, some substances such as free steroids are even not reliably measurable by plasma or blood samples and are only effectively measurable using other methods, such as the collection of saliva. These and other disadvantages of invasive procedures and techniques are of particular relevance in the field of paediatrics.

A well known non-invasive technique is the collection of saliva samples. The use of this technique allows to collect specimens of substances and/or media contained in a body fluid other than blood. The collection of oral fluid, i.e. saliva, is convenient and avoids the inherent stress of injecting a syringe or the like. This is of particular relevance when working with children or other patients susceptible to stress.

The analysis of saliva gains significance as an alternative to analyses from blood samples. Its fields of application are widely spread: Endocrinologic examinations (measuring of hormones) from saliva are established in many fields of medicine, such as gynaecology, paediatrics, sports medicine, dentistry, psychiatry and behavioural research.

Measuring medicine, drugs or narcotics is described for use in the field of pharmacology and forensic medicine. Measurement of amino acids and peptides from saliva samples is used for the diagnosis and therapy of metabolic diseases.

In some cases, the non-invasive procedure of collecting saliva samples can be carried out at home by the patient, without the need for medical supervision or participation. Some substances to be measured, e.g., steroids, are even stable for days in saliva because of the low concentration of metabolising enzymes, allowing samples that have been collected at home to be sent via mail to the medical facility.

Basically, there are two non-invasive methods known in the state of the art for collecting saliva. In the first method salvia is collected outside the patients mouth and in the second method saliva is collected inside (in-situ) the patients mouth.

According to the first method, salvia may be collected in glass tubes by expectorating saliva through a short straw into a vial. Therefore, a section of a clean, unused drinking straw or the like may be used.

According to the second method, swabs of natural products, such as cotton, or of synthetic material, e.g. polyester, are used for collecting saliva inside the patients mouth. An example for such swabs is a plain cotton roll, such as e.g. a Salivette® (Sarstedt, Nuembrecht, Germany). The method to use such a swab is to place it under the tongue of the patient for a certain time-period. The swab absorbs the salvia accumulated under the patient's tongue. Being filled with saliva, the swab can be taken out of the mouth and the salvia can be gained for examination by pressing it out of the swab into a suitable container, e.g. a glass tube.

When saliva has been collected from the patient's mouth it may then be used for measuring certain substance levels. Saliva is generally used as biological fluid for the detection of different biomarkers such as electrolytes, hormones, drugs and antibodies etc. in human and veterinary medicine. In saliva only the unbound, biologically active fraction of the hormones and drugs is measured.

The procedure of measuring and/or detecting substances, substance levels, media or the like contained in oral fluid comprises a variety of different steps of which the collection of saliva is the first. This first step is followed further by procedures such as preparing a reaction plate, tube or the like, preparing reagents and other substances needed for the detection process and the steps of analysing the saliva.

EP-B-0 863 723 discloses a method and a device for collecting a salvia sample. The device comprises a hollow body with a cavity, the hollow body having at least one perforation for the passage of salvia from the outside into the cavity. The cavity contains a removable liquid absorbing retaining body for taking up the saliva that has entered the cavity.

With the methods described above salvia is either collected via a liquid absorbing means, which has to be placed in the patients mouth for a certain time or saliva has to be expectorated through a straw into a vial.

Expectorating saliva using a straw requires the capability of the patient to perform a certain amount of active operations. These are difficult to instruct and to achieve, especially in the field of paediatrics when working with younger children or babies. Further, the use of a swab, pad or a sponge, as described above, always bears the risk of the patient swallowing the device. Furthermore, the volume of saliva which can be stored in the device is restricted and usually not sufficient for carrying out the respective analyses, particularly for a broad range of substances with a relatively low concentration in the saliva. Therefore, a number of these devices have to be used which leads to a further restriction and burden of the patient. Moreover, the above methods require a number of process steps when analysing the saliva sample.

It is therefore the object of the present invention to provide a device and a method which overcomes the existing deficiencies and disadvantages of the state of the art. It is a further object of the present invention to provide a device and a method which can easily be used with children, babies, elderly people and patients susceptible to stress and which enables a safe, effective, highly selective and sensitive way of non-invasive diagnostics and analytics. This object is achieved with the features of the claims.

According to the present invention, there is provided a device for non-invasive and in-situ concentration and detection of at least one substance contained in oral fluid, especially in saliva. The gist of the invention is that the at least one substance to be detected is bound and/or linked in-situ, i.e. in the patients oral cavity, so that no saliva has to be collected.

In a preferred embodiment of the invention, the device comprises a base element having a cavity or inner space, said base element providing a permeable connection from the outside of said base element to said cavity or inner space so that the outside of the base element is in fluid communication with said cavity. Said device is designed in a way that allows at least a part of the base element to be placed in the patients oral cavity or mouth.

The cavity or inner space of the base element preferably contains at least a part of at least one concentration element wherein the at least one concentration element has at least one binding element for binding said substance(s).

In a further preferred embodiment, said device and/or said base element comprises an opening to said cavity for locating and withdrawing said concentration element(s). Said opening is preferably closable, e.g. by means of a cover element or a lid. Said cover element is preferably detachably connected to said opening or fixed to the opening or the device, for example by means of a hinge. The cover is further preferably designed as a sliding cover. In a preferred embodiment of the invention the cover or lid closes said opening in a way that prevents the outflow of saliva. In a further preferred embodiment the opening is located in such a way, e.g. horizontally and above the patients mouth, that no lid or cover is required. The cover serves, in a further preferred embodiment of the invention, as a reaction container in which the detection and/or analysis of the saliva/substances is carried out.

The base element according to a further preferred embodiment of the present invention is made of non-rigid or flexible material. The base element is preferably made of silicon, latex or the like. Furthermore, said base element is preferably made of a material permeable to saliva. In a further preferred embodiment the base element has a plurality of holes, e.g. punched holes, pores, perforations channels and/or conduits which are permeable to saliva and which lead directly or indirectly from the outer surface of the base element to the cavity. In a preferred embodiment of the present invention said holes, pores, perforations channels and/or conduits have a diameter of about 1 mm. However, in other embodiments different diameters of larger and/or smaller scale are preferred. In further preferred embodiments, the diameters vary.

The preferred base element enables the saliva to flow from the oral cavity, i.e. the outside of the device and the base element, respectively, into said cavity or said inner space. In preferred embodiments the flow of saliva to the inside of the base element is enabled by gravity, capillary actions and/or surface tension. In other preferred embodiments chewing of the patient on the device is possible. Thereby, stimulation of salivation and supporting of the flow of saliva to the cavity of the device is achieved. In a further preferred embodiment, the base element comprises a second layer which prevents the concentration element(s) and/or the binding element(s) from being damaged or destroyed by the patient chewing on the device. This layer is permeable to saliva, e.g by being lattice shaped, and is preferably made of a material more rigid and less flexible than the material of the base element as described above. A preferred material is polyester. Further, the layer is preferably accommodated inside the base element and more preferably forms the outside of said inner cavity.

Said device and said base element, respectively, may have any form suitable to be placed in the mouth or the oral cavity of a patient, especially of a child. In preferred embodiments of the present invention said device and said base element, respectively, are substantially formed like a dummy, a teat or a cherry. In further preferred embodiments of the present invention said device and said base element, respectively, has a substantially orthodontic form. Accordingly formed devices/base elements allow a good and comfortable location of a device according to the invention in the patients mouth thereby allowing an optimal economy of space.

The concentration element(s) according to a preferred embodiment of the present invention is/are formed substantially spherically and/or lens like. In preferred embodiments the concentration element(s) have a diameter of about 5 mm or less than 5 mm. In other preferred embodiments size and geometry of the concentration element(s) may vary. The optimal size of the concentration elements depends on a number of criteria, e.g. on the number of elements to be placed in the cavity, the form of the elements, their specifity and/or their special properties. In a preferred embodiment, the diameter of said concentration element is larger than the diameter of the preferref holes, pores, perforations, channels, and/or conduits in the said base element.

At least a part of said concentration element(s) is in a preferred embodiment coated with a layer. This layer comprises, at least partially, binding elements. These binding elements may be directly coupled to the layer (for example a plastic material) or may be indirectly coupled to said layer. Coupling may be effected by covalent or non-covalent linkage. For example, the surface of the layer may be modified in a suitable way to allow coupling of the binding elements. Alternatively, the binding elements may be coupled by non-covalent forces, such as van der Waal's forces and so forth. The binding element may also be coupled via a bridge to the layer. Such a bridge may, for example, consist of avidin and streptavidin or biotin molecules. These molecules are well known in the art as binding partners that form a non-covalent but nevertheless strong linkage. If the avidin molecule is bound to the layer, then the streptavidin or biotin molecule may be coupled to the actual binding element. If the actual binding element is a protein, the biotin or streptavidin molecule may form a fusion protein with the binding element. Further methods of attaching binding elements to biotin/streptavidin are well documented in the art. The binding elements need to be specific for the analyte to be detected in the saliva. A preferred binding element is an antibody or a fragment or derivative thereof. Fragments of antibodies comprise Fab, F(ab')₂ or Fv fragments of antibodies. Derivatives of antibodies include chimeric or humanized antibodies or scFv constructs. Such antibodies or fragments thereof are well known in the art; see, for example, Harlow and Lane, "Antibodies, A Laboratory Manual", CSH press 1989, Cold Spring Harbor. The antibodies or derivatives or fragments thereof may be used to detect protein antigens, hormones or carbohydrate structures and the like in saliva. If the analyte to be detected is an antibody, then the binding element preferably is an antibody specifically recognizing that analyte antibody or an antigen known to be bound by said analyte antibody. Binding elements further include aptamers or proteins known to specifically interact with the analyte. An example of such a protein is a receptor wherein the analyte is a ligant specifically recognized by said receptor. Carbohydrate structures may also form binding elements. As is well known in the art, carbohydrate structures specifically recognize target molecules. An example of such a carbohydrate structure is the class of lectins, for example mistletoe lectine (see, for example, EP-B1 0 751 221). Binding elements may include further types of biological molecules such as lipids or glycolipids. The present invention also envisages the use of inorganic compounds as binding elements if said compounds specifically bind to the analyte.

It is preferred that the binding of the binding element to the analyte occurs with high affinity and/or high avidity.

A preferred device according to the invention is used for detection and/or analysis of at least one pre-defined substance or medium contained in saliva. A preferred device is further used with a preferred method according to the present invention. According to a further preferred method a device according to the invention is placed in a patient's mouth or oral cavity. The device stays in the patient's mouth for a certain time. In a preferred embodiment the device has to stay in the patients mouth until sufficient saliva (volume) has flown in and/or through the cavity of the device thereby contacting the concentration elements. The time required may either be predetermined or may be directly depending on the amount of contact of saliva with the concentration elements. This time, however, is, in a preferred embodiment according to the invention, shortened, e.g. when the device is designed in a way that allows a patient to chew on the device, thereby stimulating salivation and/or increasing the flow of saliva in and/or through said cavity. The device has to stay in the patients mouth for preferably 30s to 24h, more preferred for 5min to 60min or for 1min to 5min.

In a further preferred embodiment, the device comprises a fluid sensor, indicator or the like which detects or ascertains that a minimum volume of saliva got into the cavity and/or has contacted the concentration element(s).

When the concentration element(s) have been sufficiently contacted by saliva the device is taken out of the patients mouth and the concentration elements are removed from the cavity. In another preferred embodiment the concentration element may be withdrawn without taking the device out of the patients mouth. The concentration element/s is/are then placed in a reaction arrangement. At least one substance or medium being directed against the bonding structure of the at least one referred substance is added. In a further step, the qualitative or quantitative analysis of the substance is carried out.

The concentration elements may also be left within the device and the detection performed within the device, either after the device has been removed from the patient's body cavity, e.g., from the mouth, or before that removal. In this embodiment of the invention a one-step detection procedure is preferred; however, also detection methods involving several steps may be used. For instance, after removal of the device from the patient's mouth, the saliva may be removed from the cavity, e.g., using the opening (7) thereof, a labeled or unlabeled compound that is able to specifically bind the analyte is added, and the extent of specific binding is of the labeled or unlabeled compound to the analyte is measured.

In a preferred embodiment of the invention the analysis of the substance is carried out using luminescence, fluorescence, chemiluminescence, electrochemiluminescence, bioluminescence, or by detecting radioactivity. The concentration elements(s) are contacted with compounds (antibodies, antigens, nucleic acids (aptamers), carbohydrates and the like) that are coupled to a detectable label as has been mentioned in accordance with the preferred embodiments of the invention. For example, an antibody may be coupled with a fluorescent marker such as green fluorescent protein or with radioactivity such as ¹²⁵l. The compound, for example antibody, should have a different binding site as compared to the antibody used for catching the analyte and bound to the layer as has been described above. Only in cases where the analyte is a substance consisting of identical subunits, the compound used for catching the analyte and for detecting the analyte may bind to the same epitope of the analyte. A specific format of the test to be carried out includes chromatographic, radioimmunoassays (RIA), enzyme linked immunosorbent assay (ELISA), fluorescence immunoassay (FIA) and chemiluminescence immunoassay (LIA). If the analyte is a nucleic acid molecule such as a DNA molecule, detection may be effected using conventional DNA detection methods, such as polymerase chain reaction, ligase chain reaction and so forth.

The method of the invention may be used for a variety of purposes. Thus, the analytes to be detected may be indicative of diseases, such as, e.g., infection with helicobacter pylorii and other bacteria, infection with HIV and other viral pathogens, IgA insufficiency, concenital adrenal hyperplasia, and/or cystic fibrosis. Alternatively, and if the analytes to be detected are antibodies, the efficacy of an immunization may be monitored. For example, a successful immunization will give rise to an antibody response that may also be measured in the oral cavity. Furthermore, the success of a treatment with medicament may be followed up using the device of the invention. Thus, the concentration of marker molecules present in the oral cavity and indicative of the disease may be followed up using successive applications of the device of the invention. Preferred successive applications are pharmacokinetical applications, drug monitoring and/or profiling of circadian patterns e.g. of hormones.

The invention will now be described with reference to the accompanying drawings in which:
Fig. 1 shows a base element according to a preferred embodiment of the invention;
Fig. 2 shows a base element according to another preferred embodiment of the invention;
Fig. 3 shows a device according to a preferred embodiment of the invention;
Fig. 4 shows the principle of a chemiluminescence based assay; and
Fig. 5 shows the chemical transformation of acridineesters.

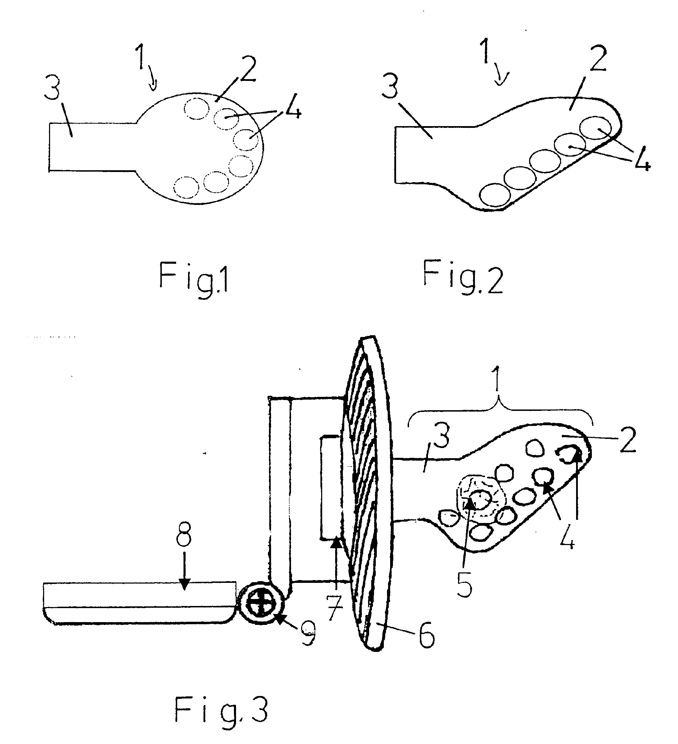

Fig. 1 shows a base element 1 according to a preferred embodiment of the invention, having a substantially cherry like formed forward portion 2 and a backward prolongation 3. The forward portion 2 of the base element comprises a number of holes 4 which lead to the cavity of the base element. These holes 4 allow saliva to flow from the outside of the element to its cavity. In the cavity of the base element 1, as described above, the at least one concentration element 5 is located. The backward prolongation 3 of the base element 1 preferably comprises an opening (not shown) that allows to withdraw said concentration element(s) 5 from said inner cavity and to place said concentration element(s) 5 within said inner cavity.

Fig. 2 shows a base element 1 according to a preferred embodiment of the invention, having a substantially orthodontic, preferably flattened, forward portion 2 and a backward prolongation 3. The base element according to fig. 2 is further preferably designed as described above with respect to fig. 1.

The base elements as shown in fig. 1 and 2 further comprise, in preferred embodiments of the invention, the features or a combination of features as described further above.

Fig. 3 shows a device according to the present invention comprising a base element 1 containing concentration elements 5, a base portion 6, an opening 7 and a cover or lid 8 being connected to the device by a hinge 9 for closing said opening 7. The base element 1 comprises at least one hole, pore, perforation and/or conduit 4 which allows saliva to get from the outside of the element 1 to its cavity and to the concentration element(s) 5. The base element 1 has a substantially orthodontic forward portion 2 and a backward prolongation 3. The backward prolongation 3 is connected to or merges with the base portion 6 which extends radially with respect to said prolongation 3. The base portion 6 comprises and opening 7 which is in connection with the opening of the base element 1 and therefore with the cavity of the base element 1. Further connected to the base portion 6 is a hinge 9 and a cover or lid 8 which allows to shut and/or seal the opening 7.

When the device is placed in a patients mouth, saliva gets into said cavity and contacts the concentration element(s) 5. As described above, these element(s) 5 is/are coated with a layer, that contains special chemical and/or biological elements. These elements bind certain substances contained in saliva. When the concentration element(s) 5 is/are taken out of the device via opening 7, these substances which are now concentrated on the surface of the concentration element(s) 5 can be detected and/or analysed.

The concentration elements may also be placed loosely within the cavity. Further, the binding elements may be placed upon the inner wall of the cavity. In this case, a separate concentration element comprising further binding elements may be added, but is not necessary for carrying out the invention.

Also comprised within the invention are devices and methods as described hereinbefore, but differing in the respect that the device does not contain separate concentration elements, but the binding elements which specifically bind to the substance that is to be concentrated and detected are located within the cavity, for instance, upon the inner cavity wall. In this embodiment of the invention, the detection is advantageously carried out without removal of said binding elements from the cavity. However, in this case, a fragment or the entire cavity wall may be removed from the remainder of the base element. Further, the fluid from which the analyte substance is concentrated need not necessarily be saliva, but may be any other fluid. A preferred fluid is saliva. Further preferred is a method wherein the device according to the invention is placed within a body cavity in order to come into contact with the body fluid that comprises the analyte to be measured. A preferred body cavity is the mouth.

The cover or lid 8 prevents the concentration element(s) 5 to fall out of and saliva to flow out of the device. In a preferred embodiment of the present invention, the cover 8 is designed to serve as a cover and as a reaction container for detecting and analysing said substances. The concentration element(s) 5 which are preferably formed as described above have, in a preferred embodiment, a diameter of about 5 mm. However, in other preferred embodiments this diameter may vary, for example depending on the number of concentration elements 5 contained in the base element 1 or depending on the size of the device which again may vary depending on its preferred use, with babies, children, elderly people, etc.

The device and the method according to the invention is preferably used for detecting, testing and/or analysing one or different substances or media contained in saliva such as gastric acid, antigens, antibodies, nuclein acids, hormones, narcotics, drugs, medecines, metabolites, etc.

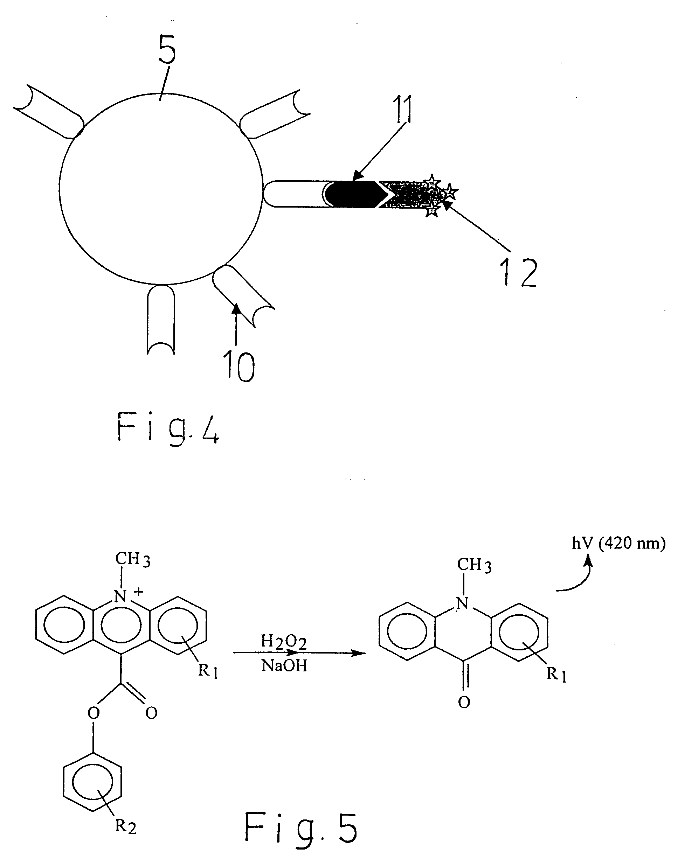

Fig. 4 shows the principle of a chemiluminescence based assay according to a preferred embodiment of the invention. A concentration element (5), being spherically shaped, which in a preferred embodiment is coated with an avidin layer comprises biotinylated specific primary antibodies (10). These antibodies bind the antigen to be detected (analyte) (11) as has been mentioned above in accordance with the preferred embodiments of the invention. The concentration element has further been contacted with a compound comprising specific secondary antibodies (12) that are coated with or coupled to acridine esters. These secondary antibodies are bound to the analyte which can thereby be detected via chemiluminescence as has been mentioned in accordance with the preferred embodiments of the invention. Fig. 5 shows, by way of example, the chemical transformation of acridineesters.

The device and the method according to the invention is preferably used with e.g. children, babies, people without control over their body or without consciousness, such as disabled, handicapped or elderly people, in psychiatry, and animals, such as e.g. trained or habituated primates. It may further be used on the side by busy people or in case of a long term analysis.

The above described preferred embodiments of the present invention are provided herein for the sake of illustration and not by way of limitation. Many further embodiments, variations, and changes to the above-described embodiments may be designed by the person of skill in the art, all without deviating from the scope of the invention. For instance, different detection methods, placement of the concentration element(s) or placement of the binding elements (for instance on the inner cavity wall), the kind of analytes to be detected, and the like, may be used. Such variations of the above-described embodiments may be designed and prepared by a person of skill in the art without undue experimentation.

The method and the device according to the present invention is/are advantageous in that it/they simplify the procedure of analysing the saliva and detecting and analysing substances contained therein. The device and the method, i.a., allow to be easily used with children and babies. Since saliva has not to be collected to test it but is bound in-situ, the device and the method reduce the number of process steps required for detecting the respective substance(s). For at least the above reasons a method and a device are provided which overcome the existing deficiencies of the state of the art and which allow an effective and efficient way to analyse saliva. The invention combines the advantages of a non-invasive pre-analysis and a highly sensitive detection method.

## Claims

1. Device for in-situ concentration of at least one substance contained in and/or detectable in a fluid, preferably in saliva, comprising
a base element (1 ) having a cavity, said base element (1 ) being in permeable connection with said cavity;
at least one binding element that binds said at least one substance.

2. Device according to claim 1, further comprising at least one concentration element (5) wherein said concentration element has at least one binding element that binds said at least one substance.

3. Device according to claim 1 or 2, further comprising an opening (7) to said cavity for placing and/or withdrawing said at least one concentration element (5).

4. Device according to any one of claims 1-4, wherein said fluid is a body fluid.

5. Device for in-situ concentration of at least one substance contained in and/or detectable in saliva, comprising
a base element (1 ) having a cavity, said base element (1 ) being in permeable connection with said cavity;
at least one concentration element (5) to be provided in said cavity, wherein said at least one concentration element (5) has at least one binding element that binds said substance(s);
said base element (1 ) further comprising an opening to said cavity for locating and withdrawing said concentration element(s) (5).

6. Device according to any one of claims 1-5, said base element (1) being made of non-rigid or flexible material.

7. Device according to one of the preceding claims, said base element (1) being made of silicon or latex.

8. Device according to any one of the preceding claims, said base element (1 ) being made of a material permeable to saliva.

9. Device according to any one of the preceding claims, wherein said base element (1) has a plurality of holes, pores, perforations and/or channels (4) which are permeable to saliva and which lead from the outer surface of said base element (1 ) to said cavity.

10. Device according to any one of the preceding claims, said holes, pores, perforations and/or channels (4) having a diameter of about 1 mm.

11. Device according to any one of the preceding claims, said device and said base element (1), respectively, being substantially formed like a dummy, a teat, or a cherry.

12. Device according to any one of the preceding claims, said device and said base element, respectively, being substantially of an orthodontic form.

13. Device according to any one of the preceding claims, said concentration element(s) (5) being formed substantially spherically and/or lens like.

14. Device according to any one of the preceding claims, said concentration element(s) (5) having a diameter of about 5 mm or less than 5 mm.

15. Device according to any one of the preceding claims, said concentration element(s) (5) being at least partly coated with a layer at least partially comprising binding elements.

16. Device according to any one of the preceding claims, said opening of said base element being closable.

17. Device according to any one of the preceding claims, said opening of said base element (1) being closable by means of a cover element (8).

18. Device according to claim 17, said cover element (8) being connectable to said device by means of a hinge (9).

19. Device according to any one of the preceding claims, wherein the substance(s) is/are selected from group of amino acids, peptides, proteins, intoxicants, drugs, medicines, nucleic acid molecules, lipids, and/or carbohydrates.

20. Device according to claim 19, wherein the amino acids, peptides, proteins, intoxicants, drugs, medicines, nucleic acid molecules, lipids, and/or carbohydrates are selected from the group of bacterial antigens, viral antigens, parasitic antigens, antibodies and/or hormones.

21. Device according to any one of the preceding claims, wherein the concentration element (5) at least partially comprises binding elements selected from the group of antibodies or the group of antigens, preferably nucleic acid molecules, carbohydrates or proteins.

22. Device according to claim 21, wherein the concentration element (5) comprises avidin and wherein the antibody/antibodies or the antigen(s) are biotinylated, bound via Protein A/G coupling, and/or coupled directly via glutaraldehyde.

23. Device according to any one of the preceding claims, wherein the substances are bound by specific binding sites of the binding elements with high affinity and/or with high avidity.

24. Method for detection of at least on substance contained in and/or detectable in a body fluid, preferably saliva, by use of a device according to any one of the preceding claims.

25. Method according to claim 24, wherein the body fluid is saliva.

26. Method for detection of at least on substance contained in and/or detectable in a body fluid, preferably saliva, comprising the steps:
placing of at least one binding element in the cavity of a basis element (1) according to any one of the preceding claims,
moisten the binding element with a body fluid,
contacting the binding element
at least one compound directed against a binding site of the substance, and
qualitative and/or quantitative determination of the extent of binding between the compound and the substance.

27. Method for detection of at least on substance contained in and/or detectable in saliva comprising the steps:
placing of at least one concentration element (5) in the cavity of a basis element (1) according to any one of the preceding claims,
moisten the concentration element with saliva,
withdrawal of the concentration element (5),
placing of the concentration element (5) in a reaction arrangement
adding of at least one element directed against a binding site of the substance, and
qualitative and/or quantitative analysis of the substance.

28. Method according to any one of claims 24 to 27, wherein the analysis of the substance is carried out by luminescence, fluorescence, chemiluminescence, electrochemiluminescence, bioluminescence, nucleic acid detection methods such as PCR, LCR or by detecting radioactivity.

29. Use of a device according to any one of claims 24 to 28 for production of a diagnostic agent for detection of amino acids, peptides, proteins, intoxicants, drugs, medicines, nucleic acid molecules, lipids, and/or carbohydrates.

30. Use of a device according to claim 23, wherein the amino acids, peptides, proteins, intoxicants, drugs, medicines, nucleic acid molecules, lipids, and/or carbohydrates are selected from the group of bacterial antigens, viral antigens, parasitic antigens, antibodies and/or hormones.

31. Device according to claim 20 or use according to claim 30, wherein the bacterial antigen is selected from the group of polysaccharides, mureins, exoproteins and/or membranebound proteins, wherein the viral antigen is selected from the group of polysaccharides and/or viral proteins, and wherein the parasitic antigen is selected from the group of polysaccharides and/or membranebound proteins.

32. Device according to claim 20 or use according to claim 30, wherein the antibody is selected from the group of IgA, IgG, IgM and IgE.

33. Device according to claim 21 or use according to claim 30, wherein the antibody is selected from the group of monoclonal antibodies or fragments or derivatives thereof, chimeric antibodies, humanized antibodies or polyclonal antibodies.

34. Device according to claim 20 or use according to claim 30, wherein the hormone is selected from the group of catecholamines, peptids, proteins, steroids, cytokins, chemokins, and/or growth factors.

35. Device according to claim 19 or 20 or use according to claim 29 or 30, wherein the amino acid is selected from the group of phenylalanine, tryptophane, tyrosine, arginine, taurine and/or other essential amino acids.

36. Device according to claim 19 or 20 or use according to claim 29 or 30, wherein the peptide is selected from the group of leptin, ghrelin, hGH, insulin, EGF, TGF, IGF and/or interleukins.

37. Device according to claim 19 or 20 or use according to claim 29 or 30, wherein the protein is selected from the group of globulins, albumins, histatins, cystatins, glycoproteins, enzymes, complement proteins and/or lipoproteins.

38. Device according to claim 19 or 20 or use according to claim 29 or 30, wherein the intoxicant is selected from the group of bacterial, viral, parasital, drugs and/or pharmaceutically active compounds.

39. Device according to claim 19 or 20 or use according to claim 29 or 30, wherein the drug is selected from the group of phenytoin, lamotrigin and/or carbamacepine.

40. Device according to claim 19 or 20 or use according to claim 29 or 30, wherein the medicine is selected from the group of antibiotics and/or antiepileptic drugs.

41. Device according to claim 19 or 20 or use according to claim 29 or 30, wherein the nucleic acid molecule is selected from the group of mRNA and/or DNA, which may be from patient, from bacterial, viral and/or parasitic origin.

42. Device according to claim 19 or 20 or use according to claim 29 or 30, wherein the lipid is selected from the group of triglycerids.

43. Device according to claim 19 or 20 or use according to claim 29 or 30, wherein the carbohydrate is selected from the group of mucopolysaccharids, lectins and/or mucins.

44. Use of a device according to any one of claims 1 to 23 or a method according to any one of claims 24 to 28 or use according to claim 29 or 30 or device according to any one of claims 31 to 43, wherein the analysis of the substance(s) is carried out by luminescence, fluorescence, chemiluminescence, electrochemiluminescence, bioluminescence, nucleic acid detection methods such as PCR, LCR or by detecting radioactivity.
